# EUROPEAN PATENT SPECIFICATION

(11) **EP 3 544 657 B1**
(45) Date of publication and mention of the grant of the patent: **20.04.2022**
(21) Application number: 17768512.0
(22) Date of filing: 03.08.2017
(51) Int. Cl.: A61M 5/142, A61M 5/145, A61M 5/20, A61M 5/31, A61M 5/14, A61M 39/24, A61F 2/30

(54) **ARTICULAR ADMINISTRATION DEVICE**
VERABREICHUNGSVORRICHTUNG FÜR GELENKE
DISPOSITIF D'ADMINISTRATION POUR DES JOINTS

(30) Priority: 28.11.2016 IT 201600120336 U; 08.02.2017 IT 201700013971
(43) Date of publication of application: 02.10.2019
(73) Proprietor: Della Bidia, Simona, 55100 Lucca (IT)
(72) Inventor: Della Bidia, Simona, 55100 Lucca (IT)
(74) Representative: Lunati & Mazzoni S.r.L.
(86) International application number: PCT/IB2017/054753
(87) International publication number: WO 2018/096408

(56) References cited:
- EP-A1- 2 022 518
- WO-A1-2011/005200
- US-A1- 2011 021 905
- US-A1- 2016 199 576

## Description

### ARTICULAR ADMINISTRATION DEVICE

The present invention relates to an articular administration device of the type specified in the preamble of the first claim.

In particular, the administration device is designed to be used to perform endo-articular infiltrations and\or infusions (for all types of joints).

Infiltration procedures for therapeutic, and more rarely diagnostic, purposes are an extremely common practice for health care professionals who take care of the musculo-skeletal system.

These procedures are used for the treatment of arthrosis or other diseases that may affect the joints, making even the simplest movements difficult and painful. Intra-articular infiltrations are performed at the joints (hip, knee, shoulder, elbow, etc.) by injecting a pharmaceutical compound suitable to restore the normal functionality of the articulation, and thus eliminate/alleviate the pain and the difficulty of movement.

They are therefore performed manually by health care professionals with the use of common syringes equipped with needles of varying length and gauge, such as hypodermic, insulin or spinal needles, or hollow needles.

Examples of an administration device are disclosed in WO2011005200, EP2022518 and US2016199576A1. US2011021905A1 discloses an administration device according to the preamble of claim 1.

The described prior art has a few major drawbacks.

A first drawback is that the infiltration must be performed periodically and only by trained health care professionals.

Therefore, over time, a patient experiences the running out of the effect of the active ingredient and hence often suffers the return of the problems he/she had before the infiltration.

As a result, apart from an immediate post-infiltration wellbeing, the patient often has no particular benefits.

He/she is thus forced to frequently turn to the health care personnel in hospitals or outpatient clinics.

Other drawbacks are that each infiltration is particularly difficult due to the high precision with which it must be carried out and because every instrument used must be sterilized.

This drawback is even more evident if one considers that often the infiltration is carried out with the assistance of ultrasound scans, CAT or other imaging devices. A no less important disadvantage is represented by the difficulty of adjusting the feasible dosage.

This disadvantage is due to the difficulty in dosing the active ingredient whose quantity, placed in the syringe and then in the patient, is evaluated by the medical staff by eye.

In addition, in case particularly high dosages are needed, several infiltrations must be performed with obvious repercussions on the patient.

In this context, the technical task underlying the present invention is to devise an articular administration device, which is capable of substantially obviating at least some of the above-mentioned drawbacks.

Within the scope of said technical task, an important object of the invention is to achieve an articular administration device, which allows infiltration and/or infusion to be performed in a precise and adjustable manner (i.e. allows the dosage and administration times to be varied).

Another important object of the invention is to provide an articular administration device capable of performing administrations that can be adjusted in time and quantity, thus producing a sustained effect in the patient.

The technical task and the specified objects are achieved by means of an articular administration device as claimed in the appended claim 1. Preferred embodiments are described in the dependent claims.

Preferred implementations are set forth in the dependent claims.

The features and advantages of the invention will be apparent from the detailed description of preferred embodiments of the invention, with reference to the accompanying drawings, in which:
**Fig. 1** shows an articular administration device according to the present invention;
**Fig. 2** shows another example of an articular administration device according to the present invention;
**Fig. 3** shows the administration device of Fig. 2 in a different moment;
**Fig. 4** shows a further configuration of the administration device according to the invention;
**Fig. 5** shows a different application of the articular administration device;
**Fig. 6** is an additional application of the articular administration device; and
**Fig. 7** shows a further application of the articular administration device.

In the present document, the measures, values, shapes and geometric references (such as perpendicularity and parallelism), when associated with terms like "about" or other similar terms such as "almost" or "substantially", are to be understood as unless measurement errors or inaccuracies due to production and/or manufacturing defects and, especially, unless a slight difference from the value, the measure, the shape, or the geometric reference with which it is associated. For example, these terms, if associated with a value, preferably indicate a difference not exceeding 10% of the value itself.

Furthermore, when used, terms such as "first", "second", "higher", "lower", "main" and "secondary" do not necessarily identify an order, a priority relationship or a relative position, but can simply be used to distinguish more clearly the different components from each other.

The measurements and the data reported in this text are to be considered, unless otherwise indicated, as carried out in the International Standard Atmosphere ICAO (ISO 2533).

With reference to the Figures, the articular administration device according to the invention is indicated as a whole by the numeral **1**.

It is adapted to be used to introduce at least one suitably fluid, and more suitably liquid, active ingredient, close to, and in particular, at an articulation. Therefore, the articular administration device 1 is adapted to be used to perform endo-articular infiltrations.

In this document, the term "active ingredient" identifies, for example, hyaluronic acid or other intra-articular medical devices, drugs or regenerative medicine aids, gene therapy, biological preparations of various kinds, solutions or suspensions.

The term liquid also includes a gel or another elastic biphasic colloidal material. The articular administration device 1, as better described hereinafter, is of the wearable type.

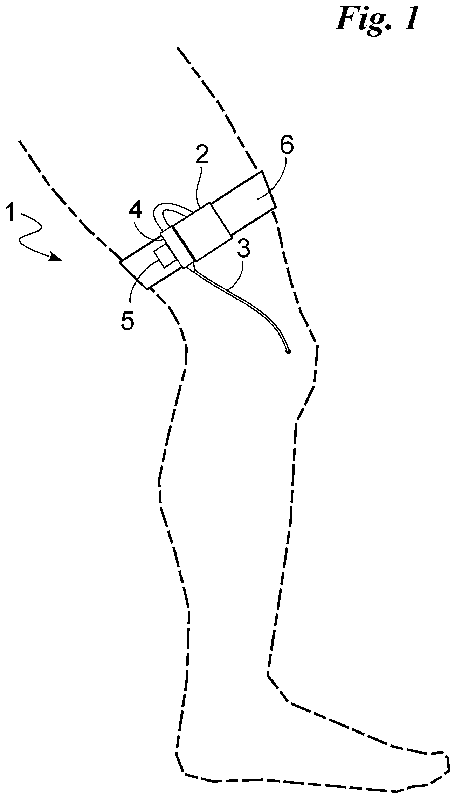

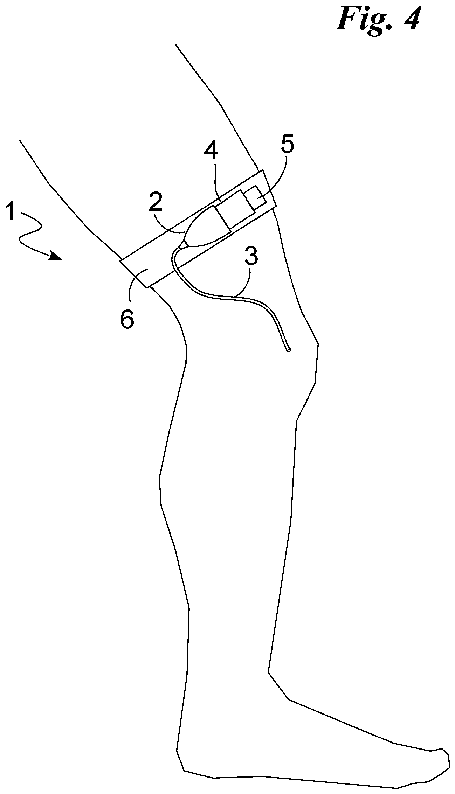

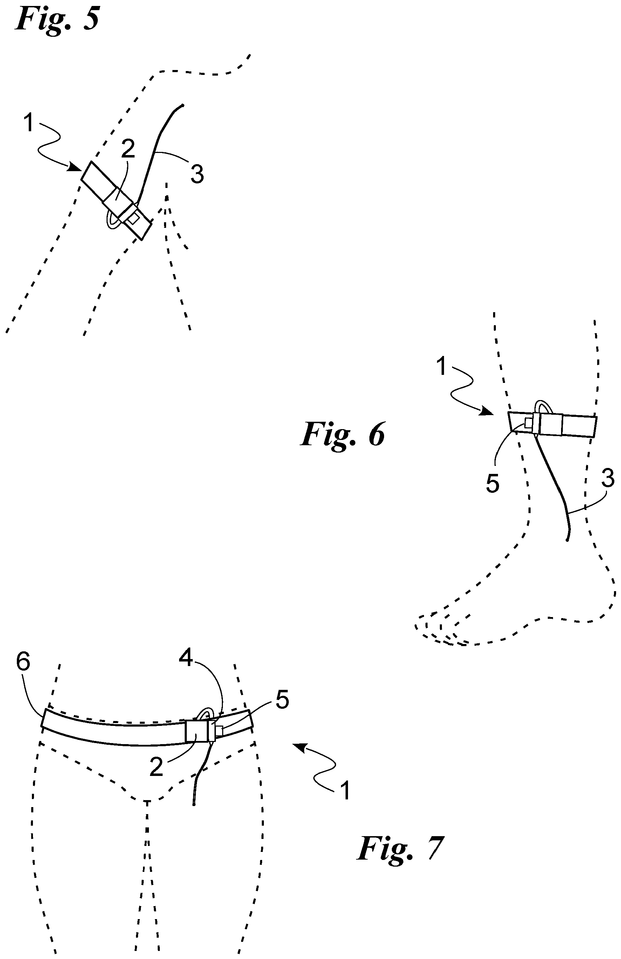

The articular administration device 1 is adapted to introduce at least one active ingredient, close to, and in particular, at an articulation, such as at the patella (knee Figs. 1 and 4), between radius and ulna (shoulder Fig. 5), between tibia and calcaneus (ankle Figs. 6), between femur and pelvis (Fig. 7).

The articular administration device 1 comprises a reservoir **2** suitable to contain at least one, usually liquid, active ingredient; at least one feeding duct **3** for the at least one active ingredient adapted to be at least partially introduced into the body at an articulation; a pump **4** designed to push (or draw) the at least one active ingredient from the reservoir 2 into the feeding duct **3** and then into the body, preferably through a control unit adapted to control the pump 4 by adjusting and controlling the flow of the at least one active ingredient introduced into the patient.

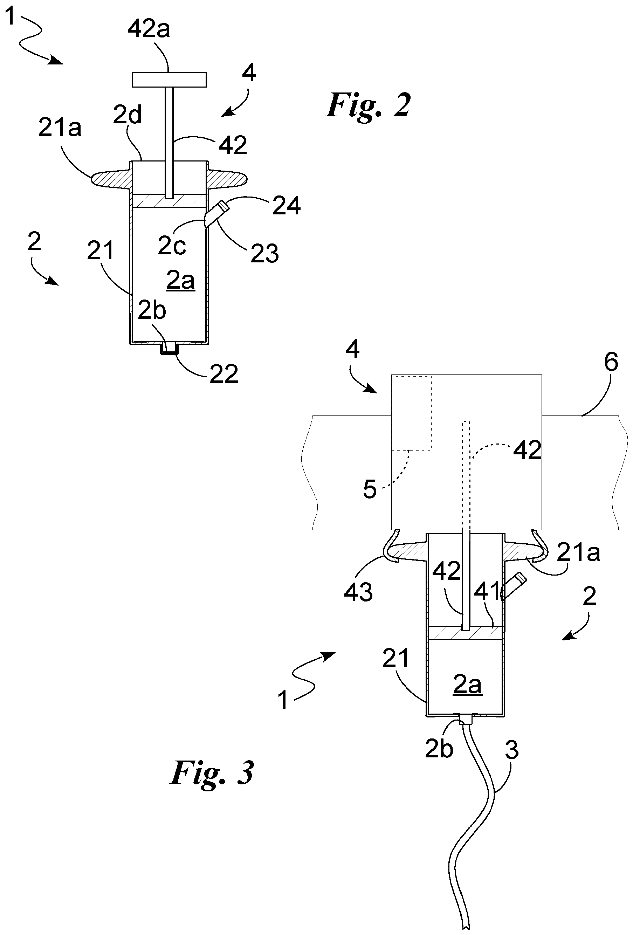

It should be noted that the reservoir 2 can be placed in fluidic through connection in a direct manner, i.e. without the interposition of components, as shown in Figs. 2, 3 and 4, or in an indirect manner, and precisely, through the pump (Fig. 1).

The control unit is adapted to control the pump 4 so that it carries out a delivery and, hence, a sustained delivery of the active ingredient. In particular, said sustained delivery may be continuous (the flow of the administered active ingredient is continuous, and preferably constant) or discontinuous (the active ingredient is administered at predefined regular intervals or at certain times).

In particular, in the case of the discontinuous sustained delivery, the articular administration device 1 can provide a clock adapted to measure the passing of time, and therefore enable the control unit to control said discontinuous delivery of the active ingredient.

The control unit (hence, the device 1) can be programmed, and therefore the device 1 can comprise means for inputting data, such as the dosage and timing of administration. The input means is identifiable either as a push-button panel, one or more knobs, or a (wired or wireless) connection to a processor (such as a computer, tablet, smartphone), which is suitably dedicated or used for this purpose through a special application.

The reservoir 2 comprises a hollow body **21** (Figs. 2 and 3) defining a collection chamber **2a** for collecting the active ingredient; and at least one opening **2b** designed to allow the introduction and\or outflow of the active ingredient.

The hollow body 21 defines a preferred extension axis and, in particular, can have a cylindrical shape, for example similar to the cylindrical body of a syringe (Figs. 2 and 3).

The hollow body 21 defines a preferred extension axis and, in particular, can have a cylindrical shape with a suitably constant cross-section. For example, the hollow body 21 is identifiable as a cylindrical body of a syringe (Figs. 2 and 3).

The opening 2b is substantially perpendicular to the preferred extension axis.

The opening 2c can have an extension smaller than that of the collection chamber 2a.

Optionally, the reservoir 2 may comprise a cap **22** detachably engageable with the opening 2b so as to close it, for example, during the filling of the reservoir.

In addition, the reservoir 2 comprises an inlet section **2c** for allowing the active ingredient to be introduced into the collection chamber 2a.

The inlet section 2c is available on the opposite side of the opening 2b with respect to the collection chamber 2a.

Said inlet section 2d can be identified as a duct **23** transverse to the preferred extension axis; and a valve **24** adapted to adjust the introduction into the collection chamber 2a through the inlet section 2c (Figs. 2 and 3).

Said valve 24 is a one-way valve, so as to prevent the active ingredient from exiting the collection chamber 2a, only allowing it to enter, preferably exclusively via a syringe. Therefore, it prevents the passage of substances both before and after being pierced by a syringe. The valve 24 is a plug valve with a pierceable membrane.

In some cases, the hollow body 21 may define an additional opening **2d** located on the opposite side of the opening 2b with respect to the collection chamber 2a.

In particular, the hollow body 21 may have, successively along the preferred extension axis, the additional opening 2d, the inlet section 2c, the collection chamber 2a, and the opening 2b, as shown in Fig. 2.

The additional opening 2d may have a section equal to that of the chamber 2a.

The feeding duct 3 defines a duct adapted to be used to place the pump 4, and in particular the reservoir 2, in fluidic through connection with the articulation, and more precisely with the inside of the articulation, so as to allow the active ingredient to be directly introduced into the region of interest. It can thus be identified as a suitably flexible cannula and/or microcatheter or other duct designed to perform this function of feeding the active ingredient.

The feeding duct 3 is connected with the collection chamber 2a through the opening 2b.

It should be noted that, in some cases, several feeding ducts 3 may be provided so as to allow the active ingredient to be administered at various points of a single articulation and/or to multiple articulations.

The pump 4 may either be a peristaltic pump, a piston pump, an elastomeric pump, suitably of the shape memory type, or a syringe infusion pump.

The peristaltic pump 4 exploits the principle of peristalsis, i.e. by creating a constriction in the feeding duct 3 which, by sliding along said feeding duct 3, pushes the active ingredient into the feeding duct 3 towards the outside and, at the same time, creates a vacuum that draws the active ingredient from the reservoir 2.

The peristaltic pump 4 may be of the rotary type and, therefore, comprise bodies compressing the feeding duct 3 (usually rollers) which, by rotating around an axis other than their own, move said constriction along the feeding duct 3.

In the case of a linear peristaltic pump 4, it differs from the rotary peristaltic pump due to the linear movement, as opposed to the rotary movement, of the bodies compressing the feeding duct 2.

Alternatively, the pump 4 may be a volumetric pump, and specifically a piston pump. Said pump 4 comprises a cylinder and a piston which, by sliding inside the cylinder, draws the active ingredient into the cylinder from the reservoir 2 and expels it therefrom introducing it into the feeding duct 3; and valves for adjusting the flow of the fluid entering and exiting the cylinder.

In a further alternative, the pump 4 may be a volumetric syringe pump comprising a manually controlled or preferably motorized plunger **41** capable of sliding at a controlled speed inside the reservoir 2 (or in an additional reservoir connected therewith), thereby adjusting the flow rate of the active ingredient introduced into the patient.

The plunger 41 is adapted to push air and\or fluid towards the opening 2b by sliding within the collection chamber 2a and then into the duct 3 suitably along the preferred extension axis. It has a section (calculated perpendicularly to the preferred extension axis) equal to that of the chamber 2a and therefore of the additional opening 2d.

The sliding of the plunger 41 is able to define at least one loading position (Fig. 2) in which the plunger 41 is positioned so that the inlet section 2c and the chamber 2a are enclosed between said plunger 41 and the opening 2b. In this position, the active ingredient can be introduced into the collection chamber 2a exclusively through the opening 2b and\or the inlet section 2c.

Preferably, in the loading position, the plunger 41 is positioned outside the reservoir 2 thanks to the additional opening 2d.

The sliding of the plunger 41 is able to define at least one working position (Fig. 3) in which the plunger 41 is between the inlet section 2c and the opening 2b. It is thus placed so as to separate the inlet section 2c from the collection chamber 2a, so that only the collection chamber 2a is enclosed between said plunger 41 and the opening 2b.

In order to allow the movement of the plunger 41, the pump 4 may comprise a thrust sleeve **42** attachable, suitably in an integral manner, to the plunger 41.

The thrust sleeve 42 may protrude from the chamber 2a through the additional opening 2d, thereby allowing the sleeve 42, and therefore the plunger 41, to be moved from the outside of the chamber 2a.

The sleeve 42 may be non-detachably, or preferably detachably (for example by interlocking or threaded coupling) constrained to the plunger 41 so as to allow the replacement of the plunger 41 and\or thrust sleeve 42.

The thrust sleeve 42 can be controlled manually, and therefore may comprise a grip **42a** (Fig. 2).

Alternatively or in addition, the movement of the thrust sleeve 42 and the plunger 41 may be motorized. Specifically, at least the movement between the reloading position and the working position, until the end stop (described below) is reached, is motorized.

To this end, the pump 4 may comprise a motor, preferably an electric motor designed to control the movement of the plunger 41 by means of an appropriate kinematic mechanism.

Specifically, the device may comprise end stop sensors capable of detecting, for example by detecting where the forward movement of the plunger 41 stops, when the plunger 41 reaches the end stop; and the control unit is adapted to control the return of the plunger 41 to a reloading position after it has reached the end stop. It should be pointed out that said function can only be performed when the duct 3 is not inserted in the articulation. It can therefore be controlled exclusively by the operator and\or by suitable means for detecting the disconnection of the duct 3 from the reservoir 2.

The end stop detects when the plunger 41 is next to the opening 2b and therefore not able to further push the active ingredient out of the collection chamber 2a. The pump 4 can be integrally (preferably detachably) connected to the reservoir 2, and specifically to the hollow body 21. It may thus comprise one or more teeth **43** adapted to engage with at least one protrusion **21a** of the hollow body 21.

The protrusion 21a extends perpendicularly to the preferred extension axis and is close to the inlet section 2b.

In order to electrically power the pump 4, the articular administration device 1 may comprise a battery **5** (Fig. 1) or other similar supply means.

The articular administration device 1 is of the wearable type and therefore comprises anchoring means **6** (Fig. 1) for attaching the device 1 to the patient, i.e. to bind the device 1 directly to the patient (for example, a limb) and\or to one or more of his/her garments.

The anchoring means 6 are designed to prevent the articular administration device 1 from moving relative to the patient, thereby ensuring its maintenance in the correct position.

The anchoring means 6 are adapted to attach at least the pump 4 and/or the reservoir 2 to the patient. They may comprise at least one length of fabric, one or more elastic band straps or one or more precisely customised garments.

The operation of the articular administration device 1, previously described in structural terms, is as follows.

The healthcare operator, after entering the active ingredient delivery parameters into the control unit, fills the reservoir with the active ingredient and attaches the device to a limb close to the articulation to be treated, thus allowing the patient to wear the articular administration device 1 and then to also use it in everyday life. Then, he/she inserts the feeding duct 3 in the articulation and commands, via the control unit, the activation of the pump 4, which starts to deliver the active ingredient according to the parameters previously inserted.

Referring to Figs. 2 and 3, initially the opening 2b of the device is obstructed by the cap 22 that is then removed. The plunger 41 is in the loading position so as to allow the active ingredient to be introduced through the inlet section 2c. Preferably, the plunger 41 is positioned outside the reservoir 2.

Once the active ingredient is inserted, the operator brings the plunger 41 into the working position by separating the inlet section 2c from the collection chamber 2a and removing the air from the collection chamber 2a.

At this point, he/she places the feeding duct 3 in the articulation and connects it to the collection chamber 2a through the opening 2b.

Then, he/she connects the plunger, more or less devoid of its thrust sleeve, to the pump 4, allowing the movement of the plunger 41, and therefore the adjustment of the administration of the active ingredient to the articulation, to be controlled in an automated way.

It should be pointed out that, if the active ingredient ends, after the separation of the duct 3 from the rest of the articular administration device 1, the control unit, appropriately as a result of a command given by the operator and\or when the plunger 41 reaches the end stop, commands the motor to place the plunger 41 in the loading position so as to allow an additional amount of active ingredient to be inserted in the collection chamber 2a through the inlet section 2c. Once the active ingredient has been introduced, the control unit, appropriately as a result of a command given by the operator, commands the motor to place the plunger 41 in the working position.

This operation can be performed while wearing the articular administration device 1.

The articular administration device 1 according to the invention achieves important advantages.

A first advantage is that the articular administration device 1 can be worn and therefore used while practising sports or any other everyday life activity.

An important advantage is that the infiltration, once the device 1 has been worn, does not require the intervention of skilled health personnel since the administration of the active ingredient is automatically performed by said articular administration device 1.

Moreover, when the reservoir 2 runs out of the active ingredient, it is sufficient to reload it to continue the treatment, therefore without having to remove the feeding duct 3 or perform other operations, which could lead to infections or other problems for the patient.

Another important advantage is that the device 1, by performing a sustained and controlled administration of the active ingredient, allows the minimum effective dosage to be used and adjusted on the basis of the clinical effect achieved, thereby reducing any adverse effects of the active ingredient due to overdose.

A further advantage is that the device 1, by performing a sustained administration of the active ingredient, allows the patient to derive maximum benefit for a much greater time than is possible today.

The invention is subject to variations falling within the scope of the inventive concept as defined by the claims. In this context, all details are replaceable by equivalent elements and the materials, shapes and dimensions may be any type of materials, shapes and dimensions.

## Claims

1. An articular administration device (1) comprising
- a reservoir (2) suitable to contain at least one active ingredient and comprising a hollow body (21) defining a collection chamber (2a) for collecting the active ingredient; and at least one opening (2b) designed to allow the introduction and\or outflow of said active ingredient from said collection chamber (2a);
- a feeding duct (3) for said active ingredient adapted to be at least partially introduced into the body at an articulation and connected with said collection chamber (2a) through said opening (2b);
- a pump (4) designed to push said active ingredient from said reservoir (2) into said feeding duct (3) and then into said articulation; and
- a control unit adapted to control said pump (4) so that it carries out the delivery of said active ingredient into said articulation; and
- said reservoir (2) comprises an inlet section (2c) for allowing an additional introduction of the active ingredient into the collection chamber (2a), **characterised in that**
the articular administration device (1) is wearable and comprises
- anchoring means for binding said articular administration device (1) directly to a patient.

2. An articular administration device (1) according to claim 1, wherein said hollow body (21) defines a preferred extension axis; and wherein said inlet section (2c) is located on the opposite side of said opening (2b) with respect to said collection chamber (2a).

3. An articular administration device (1) according to the preceding claims 1-2, wherein said reservoir (2) comprises a duct (23) transverse to said preferred extension axis and defining said inlet section (2c); and a valve (24) adapted to adjust the introduction into said collection chamber (2a) through said inlet section (2c).

4. An articular administration device (1) according to at least one of claims 1-3, wherein said pump (4) comprises a plunger (41) capable of sliding within said collection chamber (2a) along said preferred extension axis, thereby controlling the output of said active ingredient from said collection chamber (2a) through said opening (2b).

5. An articular administration device (1) according to the preceding claim, wherein said sliding of said plunger (41) is able to define at least one loading position in which said plunger (41) is positioned so that said inlet section (2c) and said collection chamber (2a) are enclosed between said plunger (41) and said opening (2b).

6. An articular administration device (1) according to the preceding claims 4 or 5, wherein said sliding of said plunger (41) is able to define at least one working position in which said plunger (41) is interposed between said inlet section (2c) and said opening (2b).

7. An articular administration device (1) according to the preceding claims 5 or 6, comprising end stop sensors capable of detecting when said plunger (41) reaches the end stop; and wherein said control unit is adapted to control the return of said plunger (41) into said loading position.

## Patentansprüche

1. Verabreichungsvorrichtung für Gelenke (1), umfassend
- einen Behälter (2), der geeignet ist, mindestens einen Wirkstoff zu enthalten und einen hohlen Körper (21) umfasst, der eine Sammelkammer (2a) des genannten Wirkstoffs und mindestens eine Öffnung (2b) definiert, die geeignet ist, das Einleiten und/oder Austreten des genannten Wirkstoffs aus der genannten Sammelkammer (2a) zu gestatten;
- eine Zuleitung (3) des genannten Wirkstoffs, die geeignet ist, an einem Gelenk mindestens zum Teil in den Körper eingeführt und an die genannte Sammelkammer (2a) über die genannte Öffnung (2b) angeschlossen zu werden;
- eine Pumpe (4), die geeignet ist, den genannten Wirkstoff aus dem genannten Behälter (2) in die genannte Zuleitung (3) zu befördern und damit in das genannte Gelenk; und
- ein Steuergerät, das geeignet ist, die genannte Pumpe (4) zum Ausgeben des genannten Wirkstoffs in das genannte Gelenk anzusteuern; und
- der genannte Behälter (2) einen Abschnitt zum Einleiten (2c) des genannten Wirkstoffs in die genannte Sammelkammer (2a) umfasst;
**dadurch gekennzeichnet, dass**
- die genannte Verabreichungsvorrichtung für Gelenke (1) tragbaren Typs ist und Mittel zum Befestigen (6) der genannten Verabreichungsvorrichtung für Gelenke (1) an einem Patienten umfasst.

2. Verabreichungsvorrichtung für Gelenke (1) nach Anspruch 1, worin der genannte hohle Körper (21) eine Achse eines bevorzugten Verlaufs definiert; und worin der genannte Abschnitt zum Einleiten (2c) im Verhältnis zu der genannten Sammelkammer (2a) an der gegenüberliegenden Seite der genannten Öffnung (2b) positioniert ist.

3. Verabreichungsvorrichtung für Gelenke (1) nach dem vorangegangenen Anspruch, worin der genannte Behälter (2) einen Kanal (23) quer zu der genannten Achse des bevorzugten Verlaufs umfasst, der den genannten Abschnitt zum Einleiten (2c) definiert; und ein Ventil (24), das geeignet ist, die Einleitungen in die genannte Sammelkammer (2a) über den genannten Abschnitt zum Einleiten (2c) zu regulieren.

4. Verabreichungsvorrichtung für Gelenke (1) nach mindestens einem der Ansprüche 2 bis 3, worin die genannte Pumpe (4) einen Kolben (41) umfasst, der geeignet ist, Einleitungen in die genannte Sammelkammer (2a) entlang der genannten Achse des bevorzugten Verlaufs gleiten zu lassen und so das Austreten des genannten Wirkstoffs aus der genannten Sammelkammer (2a) über die genannte Öffnung (2b) anzusteuern.

5. Verabreichungsvorrichtung für Gelenke (1) nach dem vorangegangenen Anspruch, worin das genannte Gleiten des genannten Kolbens (41) geeignet ist, mindestens eine Füllposition zu definieren, in der der genannte Kolben (41) so positioniert ist, dass der genannte Abschnitt zum Einleiten (2c) und die genannte Sammelkammer (2a) zwischen dem genannten Kolben (41) und der genannten Öffnung (2b) eingeschlossen sind.

6. Verabreichungsvorrichtung für Gelenke (1) nach dem vorangegangenen Anspruch, worin das genannte Gleiten des genannten Kolbens (41) geeignet ist, mindestens eine Arbeitsposition zu definieren, in der der genannte Kolben (41) zwischen dem genannten Abschnitt zum Einleiten (2c) und der genannten Öffnung (2b) eingefügt ist.

7. Verabreichungsvorrichtung für Gelenke (1) nach dem vorangegangenen Anspruch, umfassend Endanschlagsensoren, die geeignet sind, das Erreichen des Endanschlags von Seiten des genannten Kolbens (41) festzustellen; und worin das genannte Steuergerät geeignet ist, die Rückkehr des genannten Kolbens (41) in die genannten Auffüllposition anzusteuern.

## Revendications

1. Dispositif d'administration pour des joints (1) comprenant
- un réservoir (2) apte à contenir au moins une substance active et comprenant un corps creux (21) définissant une chambre de collecte (2a) de ladite substance active et au moins une ouverture (2b) apte à permettre l'introduction et/ou la sortie de ladite substance active de ladite chambre de collecte (2a) ;
- une conduite d'adduction (3) de ladite substance active apte à être au moins partiellement introduite dans le corps au niveau d'une articulation et reliée à ladite chambre de collecte (2a) à travers ladite ouverture (2b) ;
- une pompe (4) apte à pousser ladite substance active hors dudit réservoir (2) dans ladite conduite d'adduction (3) et puis dans ladite articulation ; et
- une unité de commande apte à commander à ladite pompe (4) la distribution de ladite substance active dans ladite articulation ; et
- ledit réservoir (2) comprend une section d'entrée (2c) de ladite substance active dans ladite chambre de collecte (2a) ;
**caractérisé en ce que**
- ledit dispositif d'administration pour des joints (1) est de type vestimentaire et comprend des moyens d'ancrage (6) à un patient dudit dispositif d'administration pour des joints (1).

2. Dispositif d'administration pour des joints (1) selon la revendication 1, où ledit corps creux (21) définit un axe d'extension souhaitée ; et où ladite section d'entrée (2c) est située sur le côté opposé à ladite ouverture (2b) par rapport à ladite chambre de collecte (2a).

3. Dispositif d'administration pour des joints (1) selon la revendication précédente, où ledit réservoir (2) comprend une conduite (23) transversale audit axe d'extension souhaitée et définissant ladite section d'entrée (2c) ; et une vanne (24) apte à régler les entrées dans ladite chambre de collecte (2a) à travers ladite section d'entrée (2c).

4. Dispositif d'administration pour des joints (1) selon au moins une revendication 2-3, où ladite pompe (4) comprend un piston (41) apte à faire glisser des entrées dans ladite chambre de collecte (2a) le long dudit axe d'extension souhaitée en commandant la sortie de ladite substance active de ladite chambre de collecte (2a) à travers ladite ouverture (2b).

5. Dispositif d'administration pour des joints (1) selon la revendication précédente, où ledit glissement dudit piston (41) est apte à définir au moins une position de chargement dans lequel ledit piston (41) est situé dans une position de sorte que ladite section d'entrée (2c) et ladite chambre de collecte (2a) soient placées entre ledit piston (41) et ladite ouverture (2b).

6. Dispositif d'administration pour des joints (1) selon la revendication précédente, où ledit glissement dudit piston (41) est apte à définir au moins une position de travail dans laquelle ledit piston (41) est interposé entre ladite section d'entrée (2c) et ladite ouverture (2b).

7. Dispositif d'administration pour des joints (1) selon la revendication précédente, comprenant des capteurs de fin de course aptes à détecter la position de fin de course dudit piston (41) ; et où ladite unité de commande est apte à commander le retour dudit piston (41) dans ladite position de rechargement.
